# FASCICULE DE BREVET EUROPEEN

(11) **EP 2 531 170 B1**
(45) Date de publication et mention de la délivrance du brevet: **02.07.2014**
(21) Numéro de dépôt: 11704657.3
(22) Date de dépôt: 20.01.2011
(51) Int. Cl.: A61K 8/85, A61Q 1/04, A61Q 1/06, A61Q 1/10, A61Q 1/12, A61Q 17/04, A61Q 1/02, A61Q 5/00

(54) **COMPOSITION COSMETIQUE TOPIQUE A BASE DE POLYESTER DE POLYTRIMETHYLENE ETHER GLYCOL**
KOSMETISCHE ZUSAMMENSETZUNG ENTHALTEND EINEN POLYTRIMETHYLEN ETHER GLYKOL POLYESTER
COSMETIC COMPOSITION COMPRISING A POLYESTER OF POLYTRIMETHYLENE ETHER GLYCOL

(30) Priorité: 02.02.2010 FR 1050730
(43) Date de publication de la demande: 12.12.2012
(73) Titulaire: Gattefosse S.A.S., 69800 Saint Priest (FR)
(72) Inventeur: RODIER, Jean-David, F-69100 Villeurbanne (FR)
(74) Mandataire: Denjean, Eric
(86) Numéro de dépôt international: PCT/FR2011/050099
(87) Numéro de publication internationale: WO 2011/095721

(56) Documents cités:
- EP-A2- 1 795 233
- WO-A2-2007/084636
- FR-A1- 2 795 309
- US-A1- 2008 175 875
- US-A1- 2009 092 569
- US-B1- 6 608 168

## Description

L'invention a pour objet une composition cosmétique topique contenant des polyesters de polytrimethylène ether glycol. Elle concerne plus particulièrement des compositions de maquillage telles que par exemple mascara, fond de teint, rouge à lèvres, eye liner, lipgloss, phare à joues ou à paupières, vernis à ongles mais également des compositions de protection solaire, ou encore de soin capillaire.

Le document US7427640B1 décrit des compositions cosmétiques comprenant des esters de dimère diol avec un acide monocarboxylique ou un dimère diacide. Les propriétés recherchées sont notamment le brillant (exemple 1 à 27 pour les esters d'acide monocarboxylique), les propriétés filmogènes et de résistance à l'eau lorsque le produit est appliqué sur la peau.

Le document WO2007/136586 décrit des compositions cosmétiques comprenant un polymère désigné polytrimethylène ether glycol (désigné par la suite PTEG), ne provenant pas d'une source pétrochimique et mentionné pour ses propriétés lubrifiantes. Le polytrimethylène ether glycol est un polymère de 1,3-propanediol et possède la structure suivante : H-(O-CH₂-CH₂-CH₂-)ₙ-O-H et présente un poids moléculaire compris entre 250 et 5000 g/mol. Le moyen d'obtention est décrit dans le document WO2007084636A2. Il s'agit d'une polycondensation du 1,3-propanediol en présence d'un catalyseur acide ou basique à une température comprise entre 165 et 175°C. Selon une autre caractéristique, le polytriméthylène ether glycol a un poids moléculaire compris entre 650 et 2400 g/mol.

Le document US2009/0092569A1 décrit une composition cosmétique comprenant du PTEG en tant que véhicule. Il est mentionné que le PTEG peut être obtenu à partir de diol et de diacide ou diester conformément aux enseignements du document US6 608 168 B1. Les conditions stoechiométriques et réactionnelles font que le polyester obtenu ne contient jamais de motifs acide-diol-acide.

Le document FR-A-2 795 309 décrit une composition cosmétique comprenant des esters de dimère diol avec un acide monocarboxylique ou un dimère diacide.

Le Demandeur a constaté que de manière tout à fait surprenante, l'ajout de PTEG lors de la préparation de polyesters de dimère diol avec un acide dicarboxylique ou un dimère diacide permettait de modifier les propriétés physiques et rhéologiques et notamment la viscosité du produit obtenu et donc son impact en formulation. Il a également été constaté que l'aspect brillant et le collant de la formulation lorsqu'elle était appliquée sur la peau étaient significativement améliorés.

En d'autres termes, l'invention a pour objet une composition cosmétique topique caractérisée en ce qu'elle contient un polyester comprenant les motifs ester (A-B) et (A-C) avec :
A = acide contenant au moins deux fonctions acides carboxyliques ou dimère diacide,
B = diol ou dimère diol à l'exception du polytriméthylène ether glycol
C = polytriméthylène ether glycol

Dans un mode de réalisation préféré, le polyester est exclusivement constitué de motifs (A-B) et (A-C).

Lorsque l'acide estérifié dans le motif (A-B) ou (A-C) est un acide di-carboxylique, ce dernier a la formule (I) suivante : HOOC-(CH2)n-COOH (I) dans laquelle n est un nombre entier de 1 à 20, de préférence de 3 à 16.

En pratique, l'acide dicarboxylique est choisi dans le groupe comprenant l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10décaméthylènedicarboxylique, l'acide 1,11-undécaméthylènedicarboxylique, l'acide 1,12 dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.

Dans un mode de réalisation particulier, l'acide estérifié dans le motif (A-B) ou (A-C) est un acide tricarboxylique tel que par exemple l'acide citrique ou l'acide aconitique. Ceci permet de modifier la structure du polymère et d'obtenir une forme branchée supplémentaire.

Lorsque l'acide estérifié est un dimère diacide, ledit dimère est obtenu par dimérisation intermoléculaire d'au moins un acide gras insaturé en C₈ à C₃₄, avantageusement en C₁₂ à C₂₂, de préférence en C₁₆ à C₂₀. La dimérisation de l'acide carboxylique peut éventuellement être suivie d'une hydrogénation totale ou partielle des liaisons carbone-carbone. Lorsque toutes les liaisons carbone-carbone sont hydrogénées, le dimère diacide est sous forme saturée, c'est-à-dire ne comporte aucune double liaison carbone-carbone. Dans un mode de réalisation préféré, le dimère diacide est obtenu par dimérisation intermoléculaire des acides oléique et linoléique. Le dimère diacide est alors désigné acide di-linoleique.

Lorsque B est un diol, le diol est avantageusement choisi dans le groupe comprenant :
- les diols de formule suivante (II) : HO-(CH2)n-OH (II) où n est un nombre entier de 2 à 18,
- le 12-hydroxyoctadecanol,
- le diol a la formule suivante (III) : HO-(CH2-CH2-O)n-H (III) où n est un nombre entier de 2 à 100.

Lorsque B est un dimère diol, le dimère diol est obtenu par hydrogénation d'un dimère diacide, lui-même obtenu par dimérisation intermoléculaire d'au moins un acide gras insaturé en C8 à C34, avantageusement en C12 à C22, de préférence en C16 à C20. L'étape d'hydrogénation réduit les fonctions acides afin de les transformer en fonctions hydroxyles.

Dans un mode de réalisation avantageux, le dimère diol est obtenu par hydrogénation d'un dimère diacide, lui-même obtenu par dimérisation intermoléculaire des acides oléique et linoléique.

Selon une autre caractéristique, le polytriméthylène ether glycol a un poids moléculaire compris entre 650 et 2400 g/mol.

Selon l'invention, le polyester contenu dans la composition résulte de la réaction d'estérification entre eux de A, B et C.

Le rapport molaire (B+C)/A est compris entre 0.5 et 2, avantageusement entre 0.83 et 1.2.

De même, le rapport molaire B/C est en pratique compris entre 1/99 et 95/5.

Le Demandeur a constaté qu'il obtenait des propriétés de viscosité, de brillance et de collant particulièrement intéressantes lorsque A et B était respectivement un dimère diacide obtenu par dimérisation intermoléculaire d'acide linoléique et oléique et que le dimère diol résultait de l'hydrogénation de ce dimère acide.

Il a également été constaté que les propriétés étaient encore améliorées lorsque le PTEG représentait au moins 10%, avantageusement au moins 12%, de préférence au moins 20% en poids du mélange A+B+C.

Comme déjà dit, la composition cosmétique peut être utilisée comme mascara, fond de teint, rouge à lèvres, eye liner, lipgloss, phare à joues ou à paupières, vernis à ongles, protection solaire, soin capillaire.

En fonction de la nature de la composition, le polyester représente entre 0.1% et 50% en poids de la composition cosmétique.

La composition peut contenir les adjuvants habituels dans les domaines cosmétique et dermatologique, tels que les matières grasses, les émulsionnants et co-émulsionnants, les gélifiants hydrophiles ou lipophiles, les actifs hydrophiles ou lipophiles, les conservateurs, les antioxydants, les solvants, les parfums, les charges, les filtres hydrophiles et lipophiles, les matières colorantes, les neutralisants, les agents pro-pénétrants, les parfums, les édulcorants, les agents ou polymères filmogènes, les conditionneurs.

Les quantités de ces différents adjuvants sont celles classiquement utilisées dans les domaines considérés, et par exemple de 0.01 à 30% du poids total de la composition. Ces adjuvants, selon leur nature, peuvent être introduits dans la phase grasse ou dans la phase aqueuse.

Comme matières grasses utilisables dans la composition cosmétique de l'invention, on peut utiliser les huiles minérales, les huiles d'origine animale, les huiles végétales, les huiles de synthèse (isopropyl myristate, octyldodecyl, isostearyl isostearate, decyl oleate, isopropyl palmitate), les huiles siliconées (cyclomethicone, dimethicone) et les huiles fluorées. On peut utiliser comme matières grasses des alcools gras, des acides gras, des cires et des gommes et en particulier les élastomères de silicone, la lanoline.

Comme émulsionnants et co-émulsionnants utilisables dans la composition cosmétique de l'invention, on peut citer par exemple les esters de polyglycérols et d'acides gras, les esters de saccharose et d'acides gras, les esters de sorbitan et d'acides gras, les esters d'acides gras et de sorbitan polyoxyéthylénés, les éthers d'alcools gras et de PEG, les esters de glycérol et d'acides gras, les alkyl sulfates, les alkyl éther sulfates, les alkyl phosphates, les alkyl polyglucosides, les alkyl polypentosides, les dimethicones copolyols, les dérivés de l'acide citrique tel que le glyceryl stéarate citrate, les dérivés de l'acide lactique tel que le Sodium Stearoyl Lactylate, les dérivés d'acide tartrique.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides tels que la gomme xanthane, la gomme guar, les gommes naturelles tels que la gomme de cellulose et ses dérivés, les amidons et leurs dérivés, les argiles et les copolymères d'acide 2-acrylamido-2-méthylpropane.

Comme gélifiants lipophiles, on peut citer les argiles modifiées comme les bentones, les sels métalliques d'acides gras, la silice et ses dérivés et l'éthylcellulose.

La composition cosmétique peut également contenir des actifs. Comme actifs, on peut utiliser notamment les dépigmentants, les émollients, les hydratants, les anti-séborrhéiques, les anti-inflammatoires, les anti-acnéiques, les agents kératolytiques et/ou desquamants, les agents anti-rides et tenseurs, les agents drainants, les agents anti-irritants, les agents apaisants, les amincissants tels que les bases xanthiques (caféine), les vitamines et leurs mélanges, les agents matifiants, les actifs anti-âge tel que le rétinol, les agents anti-rides, et les huiles essentielles.

Comme conservateurs utilisables selon l'invention, on peut citer l'acide benzoïque, ses sels et ses esters ; l'acide sorbique et ses sels ; les parabènes, leurs sels et esters ; le triclosan ; l'imidazolidinyl urée ; le phenoxyethanol ; la DMDM hydantoïne ; le diazolidinyl urée ; la chlorphenesin, l'acide déhydroacétique, l'alcool benzylique.

Comme antioxydants utilisables selon l'invention, on peut citer les agents chélatants tels que l'EDTA et ses sels.

Comme solvants utilisables selon l'invention, on peut citer l'eau, l'éthanol, la glycérine, le propylène glycol, le butylène glycol, le sorbitol, le propanediol.

Comme charges utilisables selon l'invention, on peut citer le talc, le kaolin, le mica, la serecite, le magnésium carbonate, l'aluminium silicate, le magnésium silicate, les poudres organiques telles que le nylon.

Comme filtres utilisables selon l'invention, on peut citer les filtres UVA et UVB classiquement utilisés tels que la benzophenone-3, le butyl methoxydibenzoyl methane, l'octocrylène, l'octyl methoxycinnamate, le 4-methylbenzylidene camphor, l'octyl salicylate, le terephthalydene dicamphor sulfanic acid, et le drométrizole trisiloxane. On citera également les filtres physiques TiO2 et ZnO sous leurs formes micrométriques et nanométriques, enrobés ou non enrobés.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Comme neutralisants utilisables selon l'invention, on peut citer la soude, la triethanolamine, l'aminomethyl propanol, l'hydroxyde de potassium.

Comme agents pro-pénétrants utilisables selon l'invention, on peut citer les alcools et glycols (éthanol, propylène glycol), l'éthoxydiglycol, les alcools et acides gras (acide oléique), les esters d'acides gras, le dimethyl isosorbide.

L'invention et les avantages qui en découlent ressortiront bien des exemples de réalisation suivants à l'appui des figures annexées.

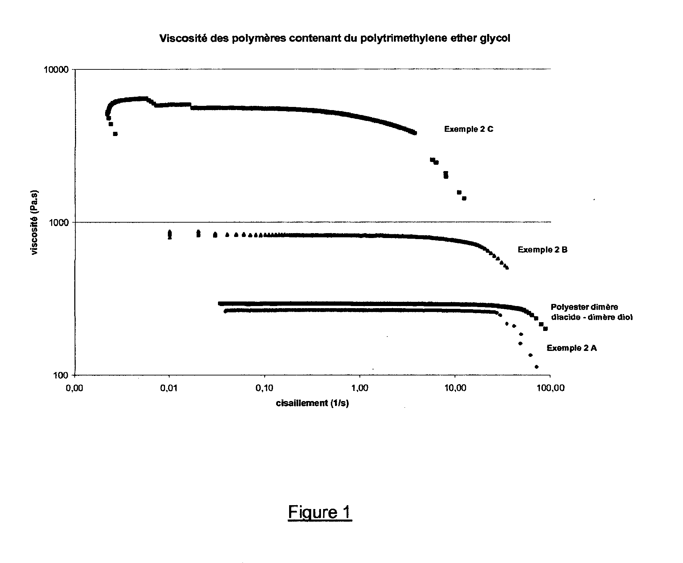

La figure 1 reflète la viscosité des polyesters de l'invention.

### Exemple 1 : protocole pour la synthèse d'un polyester

Les constituants sont introduits dans un réacteur agité et équipé d'un système de régulation de chauffage ainsi que d'un système de mise sous vide ou d'inertage à l'azote. Les produits et les quantités respectives chargées pour cet exemple sont :

| | |
|---|---|
| Dimère acide¹ di-linoléique hydrogéné | 438 g |
| Dimère diol² obtenu par hydrogénation du dimère acide ci-dessus | 200 g |
| Polytriméthylène ether glycol³ Mw=650g/mol | 362 g |

| | |
|---|---|
| ¹. Radiacid 0975 de la société Oléon ² Pripol 2033 de la société Croda ³ Cerenol® H650 de la société Dupont | |

Les réactifs sont chauffés à 200°C pendant une durée de 2 à 10 heures. La réaction est stoppée lorsque l'acidité résiduelle du milieu atteint une valeur faible. Dans l'exemple l'indice d'acide est inférieur à 2 mgKOH/g.

### Exemple 2 : variation de la proportion de polytriméthylène ether glycol dans le polyester et comparaison avec un polyester de référence

Le polymère obtenu par la réaction des constituants décrits ci-dessus possède une viscosité élevée. La viscosité augmente par l'incorporation d'une proportion croissante en polytriméthylène ether glycol.

Changement de la proportion massique en polytriméthylène ether glycol dans la fraction alcool. Le mode opératoire est le même que celui appliqué dans l'exemple 1.

| | Dimère diol | Polytriméthylène ether glycol 650 | Dimère acide |
|---|---|---|---|
| Référence | 53.3% | 0% | 46.7% |
| Exemple 2A | 41.7% | 12.6% | 45.7% |
| Exemple 2B | 30.6% | 24.7% | 44.7% |
| Exemple 2C | 20.0% | 36.2% | 43.8% |
| Exemple 2D | 0% | 57% | 42% |

Les produits obtenus sont analysés à l'aide d'un rhéomètre Rheostress 600. Les résultats de la viscosité en fonction du cisaillement sont représentés sur la figure 1. Le polymère de référence est le polyester dimère diol-dimère acide.

Comme le montrent les courbes, plus la quantité de polytriméthylène ether glycol incorporée dans le polyester est importante, plus la viscosité augmente. Le produit de l'exemple 2D est trop visqueux pour être analysé par le rhéomètre.

Le PTEG possède une structure et des groupements chimiques différents d'un diol et notamment d'un dimère diol. Pour un même rapport molaire A/(B+C) la variation du rapport C/B change significativement la structure du polymère obtenu et donc ses propriétés physiques. Lorsque le rapport C/B augmente les produits sont plus visqueux mais sont aussi plus brillants et collants lorsqu'ils sont appliqués sur la peau.

On note en outre que le polyester de l'invention donne des résultats de viscosité au moins aussi bons que le polymère de référence (voir exemple 2A).

### Exemple 3 : utilisation du produit de l'exemple 2B dans un mascara

| **Phases** | **Ingrédients** | **INCI** | **%** |
|---|---|---|---|
| **I** | Emulfree CBG | Isostearyl Alcohol (and) Butylene Glycol Cocoate (and) Ethylcellulose | 3,00 |
| | Emulium Delta | Cetyl Alcohol (and) Glyceryl Stearate (and) PEG-75 Stearate (and) Ceteth-20 (and) Steareth-20 | 3,00 |
| | Compritol 888 Ato | Glyceryl Dibehenate (and) Tribehenin (and) Glyceryl Behenate | 6,00 |
| | Lipocire A | Hydrogenated Palm Kernel Glycerides (and) Hydrogenated Palm Glycerides | 2,00 |
| | Carnauba wax | Carnauba (Corpenicia Cerifera) Wax | 3,00 |
| | Ozokérite | Ozokerite | 2,00 |
| **II** | Unipure Triple Black LC 990 | CI 77499 | 10,00 |
| | Cocoate BG | Butylene Glycol Cocoate | 8,00 |
| **III** | Eau déminéralisée | | 45,80 |
| | PVP K 120 | PVP | 1,50 |
| | Rhodicare S | Xanthan Gum | 0,30 |
| **IV** | DC 345 | Cyclopentasiloxane (and) Cyclohexasiloxane | 5,00 |
| | **Polymère de l'exemple 2B** | | **5,00** |
| | Avalure AC 120 | Acrylate Copolymer | 5,00 |
| | Glydant Plus Liquid | DMDM Hydantoin (and) Iodopropynyl Butylcarbamate | 0,40 |
| **Total** | | | 100,00 |

Apport du polymère : bonne résistance à l'eau. Il confère donc une meilleure tenue de la formule

### Exemple 4 : utilisation du produit de l'exemple 2B dans un rouge à lèvres

| **Phases** | **Ingrédients** | **INCI** | **%** |
|---|---|---|---|
| **I** | Cire de Candellila | Candelilla (Euphorbia Cerifera) Wax | 7,00 |
| | Cire de Carnauba | Carnauba (Corpenicia Cerifera) Wax | 7,00 |
| | Eutanol G | Octyldodecanol | 4,50 |
| | Hydracire S | Acacia Decurrens / Jojoba / Sunflower Seed Wax / Polyglyceryl-3 Esters | 5,00 |
| | Lipocire A | Hydrogenated Palm Kernel Glycerides (and) Hydrogenated Palm Glycerides | 6,00 |
| | **Polymère de l'exemple 2B** | | **8,00** |
| | Labrafac CC | Caprylic/Capric Triglyceride | 32,00 |
| | Cire d'abeilles | Beeswax | 2,50 |
| **II** | Prestige soft copper | Mica (and) Iron Oxides | 8,00 |
| | Unipure red LC 383 EM | (Red Iron oxide) CI 77491 | 8,00 |
| | Eutanol G | Octyldodecanol | 12,00 |
| **Total** | | | 100,00 |

### Exemple 5 : utilisation du produit de l'exemple 2B dans un eve-liner

| **Phases** | **Ingrédients** | **Nom INCI** | **%** |
|---|---|---|---|
| **I** | Tefose 2000 | PEG-6 Stearate (and) Ceteth-20 (and) Steareth-20 | 4,00 |
| | Cire de carnauba | Carnauba Wax | 4,00 |
| | Propalan T 75/20 | PEG-5 Lanolin | 2,00 |
| | **Polymère de l'exemple 2B** | | **3,00** |
| **II** | Unipur Black LC 989 | CI 77499 | 5,00 |
| | Cocoate BG | Butylene Glycol cocoate | 4,00 |
| **III** | Veegum plus | Magnesium Aluminium Silicate (and) Cellulose Gum | 1,00 |
| | Eau déminéralisée | Water | 48,60 |
| **IV** | DC 345 fluid | Cyclopentasiloxane (and) Cyclohexasiloxane | 10,00 |
| **V** | Sunshine russet | Synthetic Fluorophlogopite (and) Iron Oxyde | 10,00 |
| **VI** | Alcohol 96 | Alcohol | 5,00 |
| **VII** | Avalure AC 120 | Acrylate Copolymer | 3,00 |
| | Glydant + liquide | DMDM Hydantoin (and) Iodopropynyl. Butylcarbamate | 0,40 |
| **Total** | | | 100,00 |

Le polymère apporte une meilleure résistance à l'eau. La formule ne coulera pas à cause des larmes.

### Exemple 6 : utilisation du produit de l'exemple 2A dans un lait solaire

| Ingrédients | Nom INCI | % |
|---|---|---|
| **Phase I** | | |
| Emulium 22 | Tribehenin PEG-20 Esters | 6,00 |
| Parsol MCX | Ethylhexyl methoxycinnamate | 7,50 |
| Parsol 340 | Octocrylene | 10,00 |
| Eusolex 4360 | Benzophenone 3 | 5,00 |
| Cetiol OE | Dicaprylyl ether | 5,00 |
| Cocoate CBG | Butylene Glycol Cocoate | 5,00 |
| **Polymère de l'exemple 2A** | | **3,00** |
| Phenonip | Phenoxyethanol (and) Methylparaben (and) Ethylparaben (and) Butylparaben (and) Propylparaben | 0,80 |
| | | |
| **Phase II** | | |
| Eusolex TAVO | Titanium Dioxide (and) Silica | 7,50 |
| | | |
| **Phase III** | | |
| Eau déminéralisée | Water | 28,70 |
| Rhodicare S | Xanthan Gum | 0,30 |
| EDTA Disodique | Disodium EDTA | 0,10 |
| | | |
| **Phase IV** | | |
| Eau déminéralisée | Water | 10,00 |
| sol à 10% NaCl | Sodium hydroxyde | 5,10 |
| Eusolex 232 | Phenylbenzimidazole Sulfonic Acid | 3,00 |
| | | |
| **Phase V** | | |
| DC 1503 | Dimethicone (and) Dimethiconol | 3,00 |
| | | |
| Total | | 100,00 |

Le polymère apporte une meilleure résistance à l'eau. La protection solaire est ainsi mieux maintenue après le bain.

### Exemple 7 : utilisation du produit de l'exemple 2B dans un lipgloss

| **Phases** | **Ingrédients** | **INCI** | % |
|---|---|---|---|
| **I** | Cerabel blanc S 60V Beeswax | Beeswax | 2,00 |
| | Labrafac CC | Caprylic/Capric Triglyceride | 46,30 |
| | **Polymère de l'exemple 2B** | | **17,00** |
| | Candelilla Wax | Candelilla (Euphorbia Cerifera) Wax | 3,00 |
| | Carnauba Wax | Carnauba (Corpenicia Cerifera) Wax | 1,50 |
| | Plurol Isostéarique | Polyglyceryl-6 Isostearate | 12,00 |
| | Hydracire S | Acacia Decurrens / Jojoba / Sunflower Seed Wax / Polyglyceryl-3 Esters | 5,00 |
| | Glycérine | Glycerin | 4,00 |
| **III** | Prestige Soft Firered | Mica (and) Iron Oxides | 6,00 |
| | Prestige Dazzling Red Gold | Mica (and) Titanium Dioxide (and) Iron Oxides | 2,00 |
| | Vanilla 501104A (Firm) | | 1,20 |
| **Total** | | | 100,00 |

Le polymère apporte du brillant et surtout du collant au lipgloss, ce qui est très recherché pour la bonne tenue de celui-ci sur les lèvres.

### Exemple 8 : utilisation du produit de l'exemple 2A dans un fond de teint

| **Phases** | **Ingrédients** | **Nom INCI** | % |
|---|---|---|---|
| **I** | Hydracire S | Jojoba/Mimosa/Sunflower Seed Wax Polyglyceryl-3 Esters | 3,00 |
| | Lipocire A | Hydrogenated Palm Kernel Glycerides (And) Hydrogenated Palm Glycerides | 2,00 |
| | Cetiol CC | dicaprylyl carbonate | 9,00 |
| | Huile de jojoba | Simmondsia Chinensis Oil. | 3,00 |
| | Eutanol G | Octyldodecanol | 3,00 |
| | Labrafac CC | Caprylic/Capric Triglyceride | 4,00 |
| | **Polymère de l'exemple 2A** | | **2,00** |
| **II** | White LC 981 EM | CI 77891 | 9,10 |
| | Red LC 383 EM | CI 77491 | 0,59 |
| | Yellow LC 188 EM | CI 77492 | 2,16 |
| | Black LC 989 EM | CI 77499 | 0,15 |
| **III** | Eau déminéralisée | Water | 54,30 |
| | Surfhope C1615 | sucrose Palmitate | 1,50 |
| | Rhodicare S | Xanthan Gum | 0,30 |
| | Vanatural | Bentonite | 1,50 |
| | Viscarin 209 | Carrageenan | 0,60 |
| | Glycerine | Glycerin | 3,00 |
| **IV** | Geogard 221 | Dehydroacetic Acid (And) Benzyl Alcohol | 0,80 |
| **Total** | | | 100,00 |

Le polymère apporte une meilleure résistance à l'eau.

### Exemple 9 : préparation et utilisation de diesters de polytriméthylène ether glycol

Les produits suivants ont été préparés par estérification dans des proportions telles que deux moles d'acides gras sont utilisés pour 1 mole de PTEG.

| Exemple | Nature des acides gras mis en oeuvre | Poids moléculaire du polytriméthylène ether glycol (g/mol) mis en oeuvre | Equivalence molaire fonction acide / fonction alcool |
|---|---|---|---|
| 9A | Caprylique / caprique C8/C10 | 650 | 1/1 |
| 9B | Béhénique, C22 | 650 | 1/1 |
| 9C | Palmitique/Stéarique C16/C18 | 2400 | 1/1 |
| 9D | Béhénique, C22 | 2400 | 1/1 |

Les produits obtenus sont des diesters de polytriméthylène ether glycol.

### Exemple 10 : utilisation des produits de l'exemple 9 dans un lipgloss

Les produits obtenus dans l'exemple 9 ont été mis en oeuvre dans la formulation de lipgloss présentée ci-dessous.

| **Phases** | **Ingrédients** | **INCI** | % |
|---|---|---|---|
| **I** | Cerabel blanc S 60V Beeswax | Beeswax | 2,00 |
| | Labrafac CC | Caprylic/Capric Triglyceride | 46,30 |
| | **Diesters de l'exemple 9** | **Polytrimethylene ether glycol diester** | **17,00** |
| | Candelilla Wax | Candelilla (Euphorbia Cerifera) Wax | 3,00 |
| | Carnauba Wax | Carnauba (Corpenicia Cerifera) Wax | 1,50 |
| | Plurol Isostéarique | Polyglyceryl-6 Isostearate | 12,00 |
| | Hydracire S | Acacia Decurrens / Jojoba / Sunflower Seed Wax / Polyglyceryl-3 Esters | 5,00 |
| | Glycérine | Glycerin | 4,00 |
| **III** | Prestige Soft Firered | Mica (and) Iron Oxides | 6,00 |
| | Prestige Dazzling Red Gold | Mica (and) Titanium Dioxide (and) Iron Oxides | 2,00 |
| | Vanilla 501104A (Firm) | | 1,20 |
| **Total** | | | 100,00 |

Les formulations obtenues ont été comparées à celle de l'exemple 7.

### Résultats

| Formule de lipgloss | Diesters | Résultats de l'évaluation en comparaison avec l'exemple 7 |
|---|---|---|
| Exemple 10A | Exemple 9A | Le lipgloss obtenu n'est pas collant, le film déposé est trop fin. |
| Exemple 10B | Exemple 9B | La prise du produit est plus difficile. Le lipgloss s'étale moins bien, il est moins collant et se dépose moins. |
| Exemple 10C | Exemple 9C | Le lipgloss est ferme, voire dur, la prise est difficile. Le produit est peu collant. |
| Exemple 10D | Exemple 9D | Le lipgloss est ferme, voire dur, la prise est difficile. Le produit est peu collant. |

Compte-tenu des éléments précédents les diesters de polytriméthylène ether glycol sont moins performants que les polyesters de l'invention dans la formulation de type lipgloss.

### Exemple 11 : utilisation des produits de l'exemple 9 dans un rouge à lèvres

Les produits obtenus dans l'exemple 9 ont été mis en oeuvre dans la formulation d'un rouge à lèvres de composition suivante :

| **Phases** | **Ingrédients** | **INCI** | **%** |
|---|---|---|---|
| **I** | Cire de Candellila | Candelilla (Euphorbia Cerifera) Wax | 7,00 |
| | Cire de Carnauba | Carnauba (Corpenicia Cerifera) Wax | 7,00 |
| | Eutanol G | Octyldodecanol | 4,50 |
| | Hydracire S | Acacia Decurrens / Jojoba / Sunflower Seed Wax / Polyglyceryl-3 Esters | 5,00 |
| | Lipocire A | Hydrogenated Palm Kernel Glycerides (and) Hydrogenated Palm Glycerides | 6,00 |
| | **Diesters de l'exemple 9** | **Polytrimethylene ether glycol diester** | **8,00** |
| | Labrafac CC | Caprylic/Capric Triglyceride | 32,00 |
| | Cire d'abeilles | Beeswax | 2,50 |
| **II** | Prestige soft copper | Mica (and) Iron Oxides | 8,00 |
| | Unipure red LC 383 EM | (Red Iron oxide) CI 77491 | 8,00 |
| | Eutanol G | Octyldodecanol | 12,00 |
| **Total** | | | 100,00 |

Les formulations obtenues ont été comparées à celle obtenue en exemple 4. En plus de l'observation durant l'application du bâton de rouge à lèvres, une mesure du point de fusion (point de goutte Mettler exprimé en °C) de la formule a été réalisée.

### Résultats

| Rouge à lèvres | Produit évalué | Résultats de l'évaluation en comparaison de l'exemple 4 |
|---|---|---|
| Exemple 4 | Polyester de l'exemple 2B | Point de goutte = 68.9°C |
| Exemple 11A | Exemple 9A | Point de goutte = 62.4°C. Le bâton possède de bonnes propriétés d'étalement mais une température de fusion plus faible. Il est moins stable à la température. |
| Exemple 11 B | Exemple 9B | Point de goutte = 66.7°C. Le bâton de rouge à lèvres possède un aspect plus mat. |
| Exemple 11C | Exemple 9C | Point de goutte = 66.7°C. Le bâton de rouge à lèvres possède un aspect strié prononcé non esthétique et rédhibitoire. |
| Exemple 11D | Exemple 9D | Point de goutte = 66.3°C. Le bâton de rouge à lèvres dépose moins lors de l'application. |

On observe des différences notables entres les polymères de l'exemple 2 et les produits de l'exemple 9. Au niveau physique, on observe de manière inattendue un point de fusion plus faible pour les rouges à lèvres obtenus à partir des produits 9A, 9B, 9C et 9D qui sont des produits solides à température ambiante.

## Revendications

1. Composition cosmétique topique **caractérisée en ce qu'**elle contient un polyester exclusivement constitué des motifs ester (A-B) et (A-C) avec
A = acide contenant au moins deux fonctions carboxyliques ou dimère diacide,
B = diol ou dimère diol à l'exception du polytriméthylène ether glycol
C = polytriméthylène ether glycol ;
le rapport molaire (B+C)/A étant compris entre 0,5 et 2.

2. Composition selon la revendication 1, **caractérisée en ce que** l'acide contenant au moins deux fonctions carboxyliques est un acide di-carboxylique de formule (I) suivante : HOOC-(CH2)n-COOH (I) dans laquelle n est un nombre entier de 1 à 20, de préférence de 3 à 16

3. Composition selon la revendication 1, **caractérisée en ce que** l'acide contenant au moins deux fonctions carboxyliques est un acide tricarboxylique.

4. Composition selon la revendication 1, **caractérisée en ce que** le dimère diacide est obtenu par dimérisation intermoléculaire d'au moins un acide gras insaturé en C8 à C34, avantageusement en C 12 à C22, de préférence en C16 à C20, éventuellement suivie d'une hydrogénation totale ou partielle des liaisons carbone-carbone.

5. Composition selon la revendication 1, **caractérisé en ce que** le diol est choisi dans le groupe comprenant :
- les diols de formule suivante (II) : HO-(CH2)n-OH (II) où n est un nombre entier de 2 à 18,
- le 12-hydroxyoctadecanol,
- le diol a la formule suivante (III) : HO-(CH2-CH2-O)n-H (III) où n est un nombre entier de 2 à 100.

6. Composition selon la revendication 1, **caractérisée en ce que** le dimère diol est obtenu par hydrogénation d'un dimère diacide, lui-même obtenu par dimérisation intermoléculaire d'au moins un acide gras insaturé en C8 à C34, avantageusement en C12 à C22, de préférence en C16 à C20.

7. Composition selon la revendication 1, **caractérisée en ce que** le dimère diol est obtenu par hydrogénation d'un dimère diacide, lui-même obtenu par dimérisation intermoléculaire des acides oléique et linoléique.

8. Composition selon la revendication 1, **caractérisée en ce que** le polytriméthylène ether glycol a un poids moléculaire comprise entre 250 et 5000 et préférentiellement entre 650 et 2400 g/mol.

9. Composition selon la revendication 1, **caractérisée en ce que** le polyester est obtenu par réaction du mélange de A, B et C.

10. Composition selon la revendication 1, **caractérisée en ce que** le rapport molaire (B+C)/A est compris entre 0.83 et 1.2.

11. Composition selon la revendication 1, **caractérisé en ce que** le rapport molaire B/C est compris entre 1/99 et 95/5.

12. Composition selon la revendication 1, **caractérisée en ce que** A et B correspondent respectivement à un dimère obtenu par dimérisation intermoléculaire des acides oléique linoléique et à un dimère diol résultant de l'hydrogénation de ce dimère acide.

13. Composition cosmétique selon la revendication 1, **caractérisée en ce qu'**il s'agit d'un mascara, un fond de teint, un rouge à lèvres, un eye liner, un ligloss, un phare à joues ou à paupières, un vernis à ongles, une protection solaire, un soin capillaire.

14. Composition cosmétique selon la revendication 1, **caractérisée en ce que** le polyester représente entre 0.1% à 50% en poids de la composition cosmétique.

## Patentansprüche

1. Topische kosmetische Zusammensetzung, **dadurch gekennzeichnet, dass** sie einen Polyester enthält, der ausschließlich durch die Estereinheiten (A-B) und (A-C) gebildet ist, wobei
A = Säure mit mindestens zwei Carboxyl- bzw. Disäuredimer-Funktionen,
B = Diol bzw. Dioldimer mit Ausnahme des Polytrimethylen-Ether-Glykols,
C = Polytrimethylen-Ether-Glykol,
wobei das Molverhältnis (B+C)/A zwischen 0,5 und 2 liegt.

2. Zusammensetzung nach Anspruch 1, **dadurch gekennzeichnet, dass** die Säure mit mindestens zwei Carboxylfunktionen eine Dicarboxylsäure mit der Formel (I) HOOC-(CH2)n-COOH (I) ist, wobei n eine Ganzzahl von 1 bis 20, vorzugsweise von 3 bis 16 ist.

3. Zusammensetzung nach Anspruch 1, **dadurch gekennzeichnet, dass** die Säure mit mindestens zwei Carboxylfunktionen eine Tricarboxylsäure ist.

4. Zusammensetzung nach Anspruch 1, **dadurch gekennzeichnet, dass** das Disäuredimer durch intermolekulare Dimerisierung mindestens einer ungesättigten Fettsäure von C8 zu C34, vorteilhafterweise von C12 zu C22, vorzugsweise von C16 zu C20 erzielt wird, eventuell mit anschließender Voll- oder Teilhydrierung der Kohlenstoff-Kohlenstoff-Bindungen.

5. Zusammensetzung nach Anspruch 1, **dadurch gekennzeichnet, dass** das Diol aus der Gruppe gewählt ist, die Folgendes umfasst:
- die Diole mit der Formel (II) HO-(CH2)n-OH (II), wobei n eine Ganzzahl von 2 bis 18 ist,
- 12-Hydroxyoctadecanol,
- das Diol mit der folgenden Formel: HO-(CH2-CH2-O)n-H (III), wobei n eine Ganzzahl von 2 bis 100 ist.

6. Zusammensetzung nach Anspruch 1, **dadurch gekennzeichnet, dass** das Dioldimer durch Hydrierung eines Disäuredimers erzielt wird, das wiederum durch intermolekulare Dimerisierung mindestens einer ungesättigten Fettsäure von C8 zu C34, vorteilhafterweise von C12 zu C22, vorzugsweise von C16 zu C20 erzielt wird.

7. Zusammensetzung nach Anspruch 1, **dadurch gekennzeichnet, dass** das Dioldimer durch Hydrierung eines Disäuredimers erzielt wird, das wiederum durch intermolekulare Dimerisierung der Olein- und Linolsäure erzielt wird.

8. Zusammensetzung nach Anspruch 1, **dadurch gekennzeichnet, dass** das Polytrimethylen-Ether-Glykol ein Molekulargewicht zwischen 250 und 5000 und vorzugsweise zwischen 650 und 2400 g/mol aufweist.

9. Zusammensetzung nach Anspruch 1, **dadurch gekennzeichnet, dass** der Polyester durch Reaktion des Gemischs aus A, B und C erzielt wird.

10. Zusammensetzung nach Anspruch 1, **dadurch gekennzeichnet, dass** das Molverhältnis (B+C)/A zwischen 0,83 und 1,2 liegt.

11. Zusammensetzung nach Anspruch 1, **dadurch gekennzeichnet, dass** das Molverhältnis B/C zwischen 1/99 und 95/5 liegt.

12. Zusammensetzung nach Anspruch 1, **dadurch gekennzeichnet, dass** A und B einem Dimer, das durch intermolekulare Dimerisierung der Olein- und Linolsäure erzielt wird, beziehungsweise einem Dioldimer, das sich aus der Hydrierung dieses Säuredimers ergibt, entsprechen.

13. Zusammensetzung nach Anspruch 1, **dadurch gekennzeichnet, dass** es sich um eine Wimperntusche, eine Grundierung, einen Lippenstift, einen Eyeliner, ein Lipgloss, um Rouge oder Lidschatten, um einen Nagellack, ein Sonnenschutzmittel, eine Haarpflege handelt.

14. Zusammensetzung nach Anspruch 1, **dadurch gekennzeichnet, dass** der Polyester zwischen 0,1 Gew.-% und 50 Gew.-% der kosmetischen Zusammensetzung ausmacht.

## Claims

1. Topical cosmetic composition containing a polyester consisting exclusively of (A-B) and (A-C) type esters wherein
A = an acid containing at least two carboxylic functional groups or diacid dimer
B = a diol or dimer diol, with the exception of polytrimethylene ether glycol
C = polytrimethylene ether glycol;
the (B+C)/A molar ratio being from 0.5 to 2.

2. A composition as defined by claim 1 wherein the acid containing at least two carboxylic functional groups is a dicarboxylic acid with the following formula (I): HOOC-(CH2)n-COOH (I) in which the number n is a whole number of between 1 and 20, and preferably between 3 and 16.

3. A composition as defined by claim 1 wherein the acid containing at least two carboxylic functional groups is a tricarboxylic acid.

4. A composition as defined by claim 1 wherein the diacid dimer is obtained by intermolecular dimerization of at least one unsaturated fatty acid in C8 to C34, preferably in C 12 to C22 and especially in C 16 to C20, possibly followed by total or partial hydrogenation of the carbon-carbon bonds.

5. A composition as defined by claim 1 wherein the diol is selected from the group consisting of:
- diols with the following formula (II): HO-(CH2)n-OH (II) wherein n is a whole number between 2 and 18,
- 12-hydroxyoctadecanol,
- the diol has the following formula (III): HO-(CH2-CH2-O)n-H (III) wherein n is a whole number between 2 and 100.

6. A composition as defined by claim 1 wherein the diol dimer is obtained by hydrogenation of a diacid dimer, itself obtained by intermolecular dimerization of at least one unsaturated fatty acid in C8 to C34, preferably in C12 to C22 and especially in C 16 to C20.

7. A composition as defined by claim 1 wherein the diol dimer is obtained by hydrogenation of a diacid dimer itself obtained by intermolecular dimerization of oleic and linoleic acids.

8. A composition as defined by claim 1 wherein the polytrimethylene ether glycol has a molecular mass of between 250 and 5000, and preferably between 650 and 2400 g/mol.

9. A composition as defined by claim 1 wherein the polyester is obtained through a reaction generated by mixing A, B and C.

10. A composition as defined by claim 1 wherein the molar ratio (B+C)/A is between 0.83 and 1.2.

11. A composition as defined by claim 1 wherein the molar ratio B/C is between 1/99 and 95/5.

12. A composition as defined by claim 1 wherein A and B correspond respectively to a dimer obtained by intermolecular dimerization of oleic and linoleic acids and a diol dimer resulting from hydrogenation of this acid dimer.

13. A cosmetic composition as defined by claim 1 wherein it consists of a mascara, foundation, lipstick, eye liner, lip-gloss, blusher, eye shadow, nail polish, solar protection product or skin care product.

14. A cosmetic composition as defined by claim 1 wherein the polyester represents between 0.1% and 50% of the weight of the cosmetic composition.
